(19) Europäisches Patentamt
European Patent Office
Office européen des brevets

(11) Publication number: **0 065 774**
**B1**

(12) **EUROPEAN PATENT SPECIFICATION**

(45) Date of publication of patent specification: **03.04.85**

(51) Int. Cl.[4]: **C 07 C 29/38,** C 07 C 29/88 // C07C29/128, C07C31/24

(21) Application number: **82104481.5**

(22) Date of filing: **21.05.82**

(54) **Method for the recovery of pentaerythritol from the residual mixtures of the synthesis from acetaldehyde and formaldehyde.**

(30) Priority: **22.05.81 IT 2189181**

(43) Date of publication of application:
**01.12.82 Bulletin 82/48**

(45) Publication of the grant of the patent:
**03.04.85 Bulletin 85/14**

(84) Designated Contracting States:
**DE FR GB**

(56) References cited:
**DE-A-2 058 519**
**DE-B-1 049 841**
**GB-A- 824 551**
**GB-A-1 025 648**
**US-A-2 407 920**
**US-A-3 076 854**
**US-A-4 083 931**

(73) Proprietor: **Montedison S.p.A.**
**Patents & Licensing Dept. Foro Buonaparte, 31**
**P.O. Box 10528**
**I-20121 Milan (IT)**

(72) Inventor: **Carazzolo, Gianalvise**
**10, via Firenze**
**Castellanza(Varese) (IT)**
Inventor: **Colombo, Giancarlo**
**4, V. le Col Di Lana**
**Milano (IT)**
Inventor: **Gavella, Giulio**
**17, via Banfi**
**Imola (Bologna) (IT)**
Inventor: **Giacomuzzo, Silvano**
**31/B Via Garibaldi**
**Cassano Magnago(Varese) (IT)**
Inventor: **Gianetti, Franco**
**65, via Varese**
**Gallarate(Varese) (IT)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 065 774 B1

Courier Press, Leamington Spa, England.

**Description**

The present invention concerns a method for the recovery of pentaerythritol from residual mixtures resulting from the synthesis of pentaerythritol itself, said residual mixtures containing, together with other by-products, greater amounts of formals.

It is known from Italian Patent Publication 21222 A/78, filed in the name of the Applicant, to synthesize pentaerythritol by multiple aldolic condensation of acetaldehyde with formaldehyde and by the successive disproportioning, in the presence of aqueous sodium hydroxide, and it is known to crystallize the greatest part of the pentaerythritol produced (from the synthesis mother liquor), by then isolating separately from said mother liquor a non-negligible amount of pentaerythritol (not previously precipitated), almost stoichiometric quantities of sodium formiate and residual mixtures containing, besides minor amounts of pentaerythritol, formiate and other by-products, considerable amounts of formals. Said residual mixtures may be isolated as an alcoholic solution obtainable, for instance, according to the above cited Italian Patent Publication or according to Japanese Patent No. 15 566/77, by extraction of the formals and of the other undesired by-products from the synthesis mother liquors, using an alcohol as an extracting agent.

Said Japanese Patent Publication teaches, moreover, to remove the alcohol through evaporation and to hydrolyze the formals with an acid, in an aqueous solution.

Operating in this way, the recovery yields are not, however, very great; besides that the physical separation of the pentaerythritol from the aqueous medium thus obtained proves rather troublesome.

US—A—4 083 931 discloses a process for treating and recovering components from aldol-condensation polyol waste liquors containing major amounts of sodium formate, water and minor amounts of polyol (such as pentaerythritol) and other organic by-products. Due to the presence of large amounts of water, this process does not result in a substantial conversion of formals to the free polyol. The same is true for the process described in US—A—2 407 920.

US—A—3 076 854 and DE—A—2 058 519 are concerned with purification and recovery of polyols other than pentaerythritol. Furthermore, these references also suggest the use of large amounts of water which allow a hydrolysis reaction to proceed, but not a transacetalization as in the method of the present invention.

In its more general form, the invention concerns a method for the recovery of pentaerythritol from a residual mixture from the synthesis of the pentaerythritol itself, said mixture containing, besides other by-products, a major proportion of formals.

The method is characterized by a transacetalization of said formals, achieved by contacting the residual mixture, in a substantially anhydrous medium and in the presence of an acid, with a saturated aliphatic alcohol having from 1 to 4 carbon atoms at a temperature either equal to or greater than 60°C, but preferably comprised between 70°C and 130°C (between 70°C and 100°C when the alcohol is methanol), whereby one obtains a precipitation both of the pentaerythritol already present in the mixture in the free state, as well as of the pentaerythritol derived from the by-products (e.g. formals) by effect of said acid and alcohol, for instance according to the scheme:

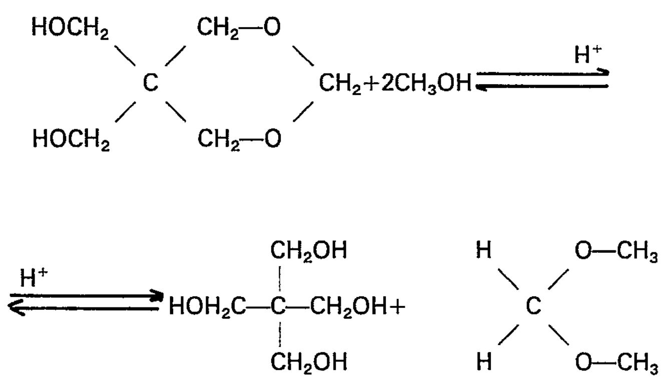


By this method are recovered considerable quantities of pentaerythritol present in the residual mixtures, either free or combined, and the recovered pentaerythritol may be separated directly, for instance, by filtering or centrifuging, from the transacetalyzation mixture in which it precipitates, due to its low solubility in alcohols.

A second advantage consists in the concomitant production of the formals of the used alcohols. If the obtained formal boils at a lower temperature than that at which boils the corresponding alcohol, then the formal itself may be easily removed from the raw transacetalyzation mixture by means of rectification.

When ethylene or propylene glycols are used as alcohol, there are obtained, in parallel, products of great value such as respectively 1,3-dioxolane and 1,3-dioxane, and when methanol is used there is obtained in parallel methylal, which is conveniently used in various fields.

A further advantage consists in the excellent solution of a delicate environmental problem connected with the disposal of discharge residues obtained otherwise.

There are different methods for realizing the invention. According to one particularly convenient form of embodiment, in no way there is added water and care will be taken that the quantity of water present be equal to or less than 10% but preferably 5% by weight with respect to the residual mixture to be trans-acetalized. The acid is chosen out of the group comprising sulphuric acid, phosphoric acid, paratoluene-sulphonic acid and the cation-exchanging resins, while the alcohol is chosen out of the group comprising methanol, ethylene glycol and 1,3-propylene glycol. The absence of water is a very important factor in as much as too high contents in water would appreciably reduce the yield of the transacetalization.

Moreover it is advisable to load the chosen acid together with the alcohol and the residual mixture to be transacetalized into a reactor surmounted by a rectification column; in this way there is in the same time achieved the transacetalization of the heavy formals and the removal of the volatile formal of the added alcohol, which has formed during the reaction, as well as the other volatile compounds present.

The dwell time in the reactor of the reaction mixture must correspond to a satisfactory degree of conversion, but at the same time it must be kept within reasonable limits. In general it would be practical to maintain said dwell time above 15 minutes and preferably keep it between 30 minutes and 5 hours or even better between 15 minutes and 3 hours.

Finally, it will be advantageous to apply by weight ratios:

$$\frac{\text{alcohol}}{\text{residual mixture to be transacetalized}}$$

comprised between 0.2 and 3, but preferably between 0.25 and 1. As far as the acid is concerned, if use is made of sulphuric acid it will be advisable to use catalytic quantities between 0.5 and 10% by weight with respect to the whole of the reaction mixture; if the acid is a different one, the quantities must be stoichiometrically equivalent. A last detail concerns the pressure which must be such as to maintain the mixture, containing the recovered pentaerythritol, in the liquid state, at the pre-established temperature; although one may also operate under vacuum, it would be advisable to keep a pressure comprised between atmospheric pressure and 5.07 bar (5 absolute atmospheres). The residual mixtures, object of this invention, in general contain, besides pentaerythritol and formals, also other compounds as for instance ethers and, in particular, small but not negligible quantities of sodium formiate, which may be conveniently removed before, by passing them over ion-exchanging resins. The invention may be applied to said mixtures either before being passed over the resin or after their passage over it, but best results are obtained when one transacetalizes mixtures pre-treated just with said resins.

The examples that will now be given hereunder serve to further illustrate the inventive idea without, however, limiting in any way the scope of the invention itself. More particularly it must be stressed that the invention may be applied to all residual mixtures containing pentaerythritol formals, however they may have been isolated.

Example No. 1

According to the method described in the Italian Patent Publication 21.222 A/78, a residual mixture is separated from the mother liquor of a pentaerythritol synthesis, said mixture containing various by-products and having the composition A reported on Table 1. A second and a third mixture, indicated by letters *B* and *C* on Table 1, were obtained by conducting the treatment of the mother liquor of the synthesis in a slightly different way, and by then pre-treating the mixtures thus obtained with cation-exchanging resins and, in the case of mixture *B*, also with anion-exchanging resins, whereby there is obtained the practically total removal of the sodium formiate still present.

441 g of the deionized mixture indicated as *B* on Table 1, were brought into contact with 147 g of methanol in the presence of 12 g of a 98% by weight sulphuric acid, thereupon bringing this reaction mixture to boiling in a flask fitted with a stirrer and surmounted by a rectification column.

It was operated under atmospheric pressure for 2 hours and 50 minutes, with a reflux ratio of 30:1, whereby the temperature in the flask passed from 75°C to 91°C, while the temperature in the head of the rectification column passed from 41°C to 58.5°C. At the end there were obtained 109 g of a distillate containing (in % by weight):

| | | | |
|---|---|---|---|
| $H_2O$ | 0.1% | $CH_3—O—CH_2—OCH_3$ | 62.35% |
| $CH_3—O—CH_3$ | 0.15% | $CH_3OH$ | 37.45% |
| $HCOO—CH_3$ | 0.05% | | |

What remained in the flask was then cooled down to room temperature and suspended for about half an hour in 190 grams of methanol, whereafter it was filtered under pressure and the crystals thereby obtained washed with further 170 grams of methanol.

After drying in a suitable oven, there were obtained 221 g of crystals containing 95.5% by weight of

pentaerythritol, which corresponded to 47.9 g of PE for each 100 g of starting mixture *B* (de-ionized mixture).

Originally the free pentaerythritol was equal only to

$$20.6 \times \frac{97.3}{100} = 20 \text{ g}$$

per 100 g of deionized starting mixture.

TABLE 1

| Components in % by weight | A | B | C |
|---|---|---|---|
| $H_2O$ | 0.9 | 2.5 | 1.5 |
| HCOONa | 3.4 | 59 ppm | 60 ppm |
| HCOOH | 0.1 | 0.02 | 1.3 |
| Free $CH_2O$ | 0.7 | 0.13 | 1.6 |
| Isobutanol | 8.8 | 0.03 | — |
| Products having the composition indicated on Table 2 | 86.1 | 97.3 | 95.6 |

TABLE 2

| Components in % by weight | A | B | C |
|---|---|---|---|
| PE(°) monomethyl ether | 0.8 | 2.4 | 0.6 |
| PE cyclic formal | 23.2 | 22.4 | 22.4 |
| Mixed $CH_3OH$/PE formal | 4.9 | 8.2 | 3.5 |
| PE dimethylether | 0.9 | 1.5 | 0.7 |
| Pentaerythritol | 12.3 | 20.6 | 18.8 |
| Dipentaerythritol | 2.1 | 1.8 | 1.5 |
| PE linear formal | 0.5 | — | 1.5 |
| Other formals | 12.3 | 7.8 | 14.5 |
| Organic compounds not identified | 43.0 | 35.3 | 36.5 |

(°)PE=pentaerythritol.

Example No. 2

441 g of the mixture marked *A* on Table 1 were brought into contact with 147 grams of methanol, in the presence of 22 g of 98% $H_2SO_4$, according to the operative conditions followed in the preceding example; thereby in the flask the temperature passed from 71°C to 88°C and, in the head of the column, it rose from 40° to 64°C. Thereby were gathered 110 g of a distillate containing (in % by weight):

| | | | |
|---|---|---|---|
| $H_2O$ | 0.3% | $CH_3O—CH_2—OCH_3$ | 47.9% |
| $CH_3OCH_3$ | 0.2% | $CH_3OH$ | 39.3% |
| $H—COO—CH_3$ | 12.3% | | |

From the content that had remained in the flask, by operating as in Example 1, there were extracted 121 g of crystals containing 86.0% by weight of PE and 8% by weight of sodium sulphate, what corresponds to 23.6 g of PE every 100 g of starting mixture *A*. It must be pointed out that originally the free pentaerythritol equalled only

$$12.3\times\frac{86.1}{100}=10.6 \text{ g/100 g}$$

of starting mixture *A*.

Example No. 3:
399 g of the deionized mixture indicated on Table 1 as mixture *B*, were poured into a flask containing 210 g of isobutanol, in the presence of 12 g of 98% $H_2SO_4$.

This reaction mixture was thereupon heated for 3 hours by total reflux, thereby stabilizing the temperature around 103°C. The mixture was then cooled down to room temperature, filtered under pressure and finally washed with 100 g of methanol thereby obtaining 121 g of crystals containing 94.5% by weight of PE, that is 28.7 g of PE per 100 g of starting mixture *B*, against the 20 grams of free PE that were originally present in each 100 g of mixture *B*. It should be noted the considerably lower yield with respect to the yield that may be obtained using methanol.

Example No. 4:
396 g of the de-ionized mixture indicated with *B* on Table 1, were brought into contact with 297 g of 1,3-propylene glycol, in the presence of 6 g of 98% $H_2SO_4$, operating as in example 1, under an absolute pressure of 107 mbar (80 mmHg) and with a reflux ratio of 20:1, whereby the temperature in the flask passed from 110° to 130°C while in the head of the rectifying column the temperature passed from 25° to 35°C.

After 2.5 hours there were gathered 155 g of a distillate containing (in % by weight):

| | | | |
|---|---|---|---|
| $H_2O$ | 2.5% by weight | 1,3-dioxane | 82.4% |
| $CH_3OH$ | 11.7% by weight | other compounds | 3.4% |

The reaction mixture was thereupon cooled down to room temperature, suspended in 1000 g of methanol for about half an hour and finally filtered under pressure.

The crystals thus obtained were then suspended in 100 g of methanol, filtered again and then washed with further 100 g of methanol.

Thereby were obtained 203 g of crystals containing 97% by weight of pentaerythritol, that is, 49.7 g of PE/100 g of initial de-ionized mixture, against the 20 g that were originally present.

Example No. 5:
800 g of the mixture indicated on Table 1 by *C*, were brought into contact, in a carbon steel autoclave with thermostatically controlled heating and fitted with a stirrer, with 325 g of $CH_3OH$, and the whole was then brought up to a temperature of 87°C; the pressure amounted to about 1.7 bar. The mixture was thereupon additioned with 25 g of $H_2SO_4$ at a 98% by weight concentration, dissolved in 100 g of methanol.

During the first 15 minutes there was observed an autogenous and gradual rise in pressure up to about 2.5 bar.

At this point one started to exhaust the vapors that had formed during this first part of the reaction, vapors that contained mainly methylale and methanol, and a liquid restoring quantity of 760 g/hour of methanol, equal to about the methanol removed in the vapor phase, was fed into the reactor whereby the pressure rose abruptly again to the level of about 1.3 bar. During the test the temperature was maintained at 87°C.

2.25 hours after the addition of sulphuric acid, the mixture was rapidly cooled down to room temperature, discharged from the autoclave and then filtered under pressure. The crystals thus obtained were then washed with 200 g of methanol. After drying in an oven, there were obtained 283 g of crystals containing 94.0% by weight of pentaerithritol, what corresponds to 33.3 g of PE/100 g of initial Mixture *C* (deionized mixture).

Originally the free pentaerythritol was equal only to

$$18{,}8\times\frac{95.6}{100}=18\ g$$

per 100 g of initial deionized mixture.

Example No. 6:

300 g of the mixture indicated as "C" in Table 1, were mixed with 70 g of methanol and with 280 g of a strongly acidic cation exchange resin (Kastel C-331-P, produced by the Applicant).

The procedure of Example 1 was repeated except for the periodical liquid reintegration of methanol, in order to keep constant its content in the reaction mixture. The reaction was carried out for 9 hours with a reaction temperature of 72°C (±2°C) and with a head distillation temperature of from 50° to 63.5°C.

After 6 hours and 12 minutes, 156 grams of distillate were obtained. The composition of the distillate is given in the following table (weight percent):

| | | | |
|---|---|---|---|
| $H_2O$ | 0.45 | $CH_3OCH_2OCH_3$ | 36.80 |
| $CH_3OCH_3$ | 0.45 | $CH_3OH$ | 61.75 |
| $HCOOCH_3$ | 0.55 | | |

A second distillate, collected between the first one and the end of the distillation, weighed 57 g, and contained (weight percent):

| | | | |
|---|---|---|---|
| $H_2O$ | 0.30 | $CH_3OCH_2OCH_3$ | 10.62 |
| $CH_3OCH_3$ | 0.50 | $CH_3OH$ | 88.58 |
| $HCOOCH_3$ | $<0.05$ | | |

At the end of the reaction, the reactor and its contents were cooled down to a temperature of 25°C and said contents were mixed together with 100 g of methanol. The ion exchanger was then removed from the reaction mixture by wet screening on a 60 mesh steel sieve, and then washed with 200 grams of methanol; the latter one and the slurry not retained by the screening, were mixed together and filtered under pressure. The cake was then washed with 100 g of methanol and then dried in an oven, whereby there were obtained 140 g of a solid whose pentaerythritol content was found to amount to 97.5% by weight; this means that 45.5 parts of pentaerythritol were obtained from 100 parts of starting mixture C.

Originally the "free" pentaerythritol was only

$$18.8\times\frac{95.6}{100}=18\ \text{parts}$$

by 100 parts of starting mixture C.

**Claims**

1. A method for the recovery of pentaerythritol from a residual mixture of the synthesis of pentaerythritol from acetaldehyde and formaldehyde, said mixture containing, besides other by-products, major amounts of formals, characterized in that said formals are transacetalized by contact of said residual mixture, in a substantially anhydrous medium and in the presence of an acid, with a saturated aliphatic alcohol having from 1 to 4 carbon atoms at a temperature equal to or higher than 60°C and for a time greater than 15 minutes, whereby one obtains a precipitation both of the pentaerythritol already present in said mixture in the free state as well as of the pentaerythritol derived from the by-products (predominantly formals) by effect of said acid and alcohol.

2. The method of claim 1, wherein the transacetalization is carried out at a temperature of between 70 and 130°C and for a time between 30 minutes and 5 hours, preferably between 0.5 and 3 hours.

3. The method of claim 1 or 2, wherein the amount of water present is equal to or lower than 5% by weight with respect to the residual mixture.

4. The method of any of claims 1—3, wherein said alcohol is selected from methanol, ethylene glycol and 1,3-propylene glycol.

5. The method of any of claims 1—4, wherein said acid is selected from sulphuric acid, phosphoric acid, paratoluene sulphonic acid and cation-exchange resins.

6. The method of any of claims 1—5, wherein the pressure is between 1.01 and 5.07 bar (1 and 5 absolute atmospheres).

7. The method of any of claims 1—6, wherein the weight ratio of alcohol to the residual mixture to be transacetalized is between 0.2 and 3, preferably between 0.25 and 1.

8. The method of any of claims 1—7, wherein said acid is sulphuric acid and the quantity of acid is between 0.5 and 10% by weight with respect to the whole reaction mixture.

9. The method of any of claims 1—8, wherein said residual mixture is deionized, before the transacetalization, by passing it through ion-exchange resins.

10. The method of any of claims 1—9, wherein said residual mixture contains, besides other by-products, at least 20%, preferably 30% by weight of formals.

11. The method of any of claims 1—10, wherein said transacetalization is carried out by contact of said residual mixture, optionally deionized, in the presence of sulphuric acid, with a quantity of methanol of between 0.25 and 1 kg/kg of said residual mixture at a temperature of between 70 and 100°C at a pressure of between 1.01 and 3.04 bar (1 and 3 absolute atmospheres) and for a time of between 30 minutes and 3 hours.

**Patentansprüche**

1. Verfahren zur Gewinnung von Pentaerythrit aus einem Rückstandsgemisch der Synthese von Pentaerythrit aus Acetaldehyd und Formaldehyd, welches Gemisch neben anderen Nebenprodukten größere Mengen von Formalen enthält, dadurch gekennzeichnet, daß die Formale durch Kontakt des Rückstandsgemisches in einem im wesentlichen wasserfreien Medium und in Gegenwart einer Säure mit einem gesättigten aliphatischen Alkohol mit 1 bis 4 Kohlenstoffatomen bei einer Temperatur von gleich oder höher als 60°C und über eine Zeit von mehr als 15 Minuten transacetalisiert werden, wobei man durch Wirkung der Säure und des Alkohols einen Niederschlag sowohl des bereits in dem Gemisch in freiem Zustand vorhandenen Pentaerythrits als auch des von den Nebenprodukten (überwiegend Formalen) abgeleiteten Pentaerythrits enthält.

2. Verfahren nach Anspruch 1, worin die Transacetalisierung bei einer Temperatur zwischen 70 und 130°C und über eine Zeit zwischen 30 Minuten und 5 Stunden, vorzugsweise zwischen 0,5 und 3 Stunden, durchgeführt wird.

3. Verfahren nach Anspruch 1 oder 2, worin die vorhandene Wassermenge gleich oder niedriger ist als 5 Gewichtsprozent, bezogen auf das Rückstandsgemisch.

4. Verfahren nach irgendeinem der Ansprüche 1 bis 3, worin der Alkohol ausgewählt ist unter Methanol, Ethylenglykol und 1,3-Propylenglykol.

5. Verfahren nach irgendeinem der Ansprüche 1 bis 4, worin die Säure ausgewählt ist unter Schwefelsäure, Phosphorsäure, p-Toluolsulfonsäure und Kationenaustauscherharzen.

6. Verfahren nach irgendeinem der Ansprüche 1 bis 5, worin der Druck zwischen 1,01 und 5,07 bar (1 und 5 absoluten Atmosphären) beträgt.

7. Verfahren nach irgendeinem der Ansprüche 1 bis 6, worin das Gewichtsverhältnis von Alkohol zu dem zu transacetalisierenden Rückstandsgemisch zwischen 0,2 und 3, vorzugsweise zwischen 0,25 und 1 beträgt.

8. Verfahren nach irgendeinem der Ansprüche 1 bis 7, worin die Säure Schwefelsäure ist und ihre Menge zwischen 0,5 und 10 Gewichtsprozent, bezogen auf das gesamte Reaktionsgemisch, beträgt.

9. Verfahren nach irgendeinem der Ansprüche 1 bis 8, worin das Rückstandsgemisch vor der Transacetalisierung entionisiert wird, indem man es durch Ionenaustauscherharze leitet.

10. Verfahren nach irgendeinem der Ansprüche 1 bis 9, worin das Rückstandsgemisch neben anderen Nebenprodukten mindestens 20, vorzugsweise 30 Gewichtsprozent Formale enthält.

11. Verfahren nach irgendeinem der Ansprüche 1 bis 10, worin die Transacetalisierung durch Kontakt des gegebenenfalls entionisierten Rückstandsgemisches in Gegenwart von Schwefelsäure mit einer Methanolmenge von zwischen 0,25 und 1 kg/kg Rückstandsgemisch bei einer Temperatur zwischen 70 und 100°C und einem Druck zwischen 1,01 und 3,04 bar (1 und 3 absoluten Atmosphären) für eine Zeit zwischen 30 Minuten und 3 Stunden durchgeführt wird.

**Revendications**

1. Procédé de récupération du penta-érythritol contenu dans le mélange résiduel résultant de la synthese du penta-érythritol à partir d'acétaldéhyde et de formaldéhyde, ce mélange contenant outre les sous-produits des quantités majeures de formal, caractérisé en ce que lesdits formals sont transacétalisés par contact du mélange résiduel, dans un milieu pratiquement substantiellement anhydre et en présence d'un acide, avec un alcool aliphatique saturé comprenant 1 à 4 atomes de carbone, à une température égale ou supérieure à 60°C et pendant une durée de temps supérieure à 15 minutes de faon à ce que l'on obtienne la précipitation à la fois du penta-érythritol déjà présent dans ledit mélange à l'état libre ainsi que du penta-érythritol résultant des sous-produits (formals de faon prédominante) par l'effet de la réaction desdits acide et alcool.

2. Le procédé selon la revendication 1, caractérisé en ce que la transacétalisation est effectuée à une

température comprise entre 70° et 130°C et pendant une durée de 30 minutes à 5 heures, de préférence de 0,5 à 3 heures.

3. Le procédé selon la revendication 1 ou 2, dans lequel la quantité d'eau présente est égale ou inférieure à 5% en poids par rapport au mélange résiduel.

4. Le procédé selon une quelconque des revendications 1 à 3, dans lequel ledit alcool est choisi parmi méthanol, éthylèneglycol et 1,3-propylèneglycol.

5. Le procédé selon une quelconque des revendications 1 à 4, dans lequel ledit acide est choisi parmi acide sulfurique, acide phosphorique, acide paratoluènesulfonique et les résines échangeuses de cations.

6. Le procédé selon une quelconque des revendications 1 à 5, dans lequel la pression est comprise entre 1,01 et 5,07 bars (1 et 5 atmosphères absolus).

7. Le procédé selon une quelconque des revendications 1 à 6, dans lequel le rapport pondéral alcool/mélange résiduel à transacétaliser est compris entre 0,2 et 3, et de préférence est compris entre 0,25 et 1.

8. Le procédé selon une quelconque des revendications 1 à 7, dans lequel ledit acide est l'acide sulfurique et la quantité d'acide est comprise entre 0,5 et 10% en poids par rapport à la totalité du mélange réactionnel.

9. Le procédé selon une quelconque des revendications 1 à 8, dans lequel ledit mélange résiduel est déminéralisé, préalablement à la transacétalisation, par passage sur des résines échangeuses d'ions.

10. Le procédé selon une quelconque des revendications 1 à 9, dans lequel ledit mélange résiduel contient, outre des sous-produits, au moins 20%, de préférence 30% en poids, de formals.

11. Le procédé selon une quelconque des revendications 1 à 10, dans lequel ladite transacétalisation est effectuée par contact dudit mélange résiduel, éventuellement déminéralisé, en présence d'acide sulfurique avec une quantité de méthanol comprise entre 0,25 et 1 kg/kg dudit mélange résiduel, à une température comprise entre 70° et 100°C, sous une pression de 1,01 et 3,04 bars (1 et 3 atmosphères absolus) pour une durée comprise entre 30 minutes et 3 heures.